# EUROPEAN PATENT SPECIFICATION

(11) **EP 0 395 751 B1**
(45) Date of publication and mention of the grant of the patent: **28.07.1993**
(21) Application number: 89912232.9
(22) Date of filing: 20.10.1989
(51) Int. Cl.: A61M 5/158, A61M 39/02

(54) **INFUSION CANNULA**
INFUSIONSKANÜLE
CANULE POUR PERFUSIONS

(30) Priority: 20.10.1988 DK 5858/88
(43) Date of publication of application: 07.11.1990
(73) Proprietor: CODAN Medizinische Geräte GmbH & Co. KG, D-23735 Lensahn (DE)
(72) Inventor: KNUDSEN, Soren, DK-3070 Snekkersten (DK)
(74) Representative: Stellinger, Jens-Holger
(86) International application number: DK8900247
(87) International publication number: WO9004421

(56) References cited:
- EP-A- 0 015 443
- SE-B- 419 163
- SE-C- 211 721
- US-A- 4 231 367

## Description

The invention relates to an infusion cannula having wings for fastening to a patient and at least one valve stub forming an injection channel with a valve cap being rotatable around two pivots extending in continuation of each other perpendicular to a plane being defined by the longitudinal axes of the cannula and the valve stub, whereby, through the valve stub, injections can be made into the patient during infusion.

Such an infusion cannula is known from US Patent 3,802,433, where the valve stub is arranged diametrically opposed to the wings, which provide a support surface to the patient, and which serve to the fastening to the patient. This results in the valve stub and it's cap protruding from the infusion cannula, whereby the valve cap at the movements of the patient can be pushed off with a contamination as a result. Further such infusion cannula requires a pull perpendicular to the logitudinal direction of the infusion cannula using two hands, when the cap is removed during the preparation of an injection, as, during the removal of the cap, for the patient's sake or eventually a loosening of the cannula, the cannula should be held back

From DE Publication 216 01 993 an infusion cannula with a socket case is known, to which, supposedly through a cone, a connecting piece is fastened being equipped with a transverse valva stub. This valve stub is upwardly open, and so it is easily contaminated.

An infusion cannula is known from the DE Publication No. 30 31 242, where the valve stub is provided with a cap, which is hinged along the one side of the valve stub. For the opening of this cap it is necessary exert a pull perpendicular to the logitudinal direction of the cannula, which either causes inconveniences to the patient in the form of pain, or two hands are required for handling the cap. This infusion cannula is not secure from the cap being pushed off as a result of the patient's movements, and so, also here, there is an unacceptable large risk for a contamination of the injection channel.

In the following, at the disclosure of the infusion cannula, the term 'proximal' is used, which in relation to the infusion cannula means that part of the infusion cannula, which at normal use, is closest to the operational staff, whereas the opposite end of the infusion cannula is referred to as the 'distal' end. This means, at the introduction of an infusion cannula into a patient, that coupling means will be in the proximal end of the infusion cannula, whereas the point of the infusion cannula constitutes the distal end.

Infusion cannulas are intended for use at infusion of fluid into patients. To this purpose a hypodermic needle is put on the distal end of the infusion cannula, which can be equipped with a soft cover. After the puncture of skin and vein the needle can be pulled out again, whereupon the soft cover stays embedded in the vein. The infusion cannula is fixed to the skin through the wings, and containers with the infusion fluid is connected to the proximal end of the infusion cannula.

If medicin is to be administered or if a physical examination shall be made, during which contrast substances or sedating compositions are to be injected or the like, it is desirable, because of the patient, to avoid further inconveniences caused by introducing hypodermic needles. To this purpose, the valve stub fixed to the infusion cannula is used, whereby the valve stub can be constructed as a valve stub known from the state of the art, eventually with a cap and a not shown membrane or the like means in order to prevent leakage of the infusion fluid through the valve stub. The infusion cannula is usually fastened to the back of the hand, where it is easily accessible for the nursing staff. If the patient's hand or arm are not fixed, the infusion cannula on the back of the hand is, however, exposed to collisions with various hard objects in the proximity of the patient. In that way there is a risk, that the patient pushes off the valve cap from the valve stub, and there is a risk for a comtamination of the interior of the valve stub.

To infusion cannulas being designed as mentioned above there is connected that considerable drawback, that it is not possible to prepare an injection through the infusion cannula by the use of only one hand. This is, however, desirable for the nursing staff, as an injection should be given immediately after the filling of the syringe without having to put the syringe away during the preparation of the injection through the infusion cannula causing a risk for comtamination of the injection cannula being introduced in the infusion cannula fixed to the patient. Further, those infusion cannulas being equipped with a cap are not sufficiently secured against an unintended opening of the cap caused by the movements of the patient, what increases the risk for contamination of the injection channel.

An object for the present invention is consequently to secure an one hand operation of the cap on the valve stub securing the cap against unintended opening.

This is accomplished according to the invention in an infusion cannula as mentioned above whereby the pivots are provided by a spindle arranged on the infusion cannula on a level with the valve stub, and the valve cap has a skirt surrounding the valve stub having a height approximately corresponding the height of the valve stub and has an opening on the proximal side being so dimensioned, that the valve stub can pass through it, when the valve cap is rotated, and the skirt on the sides of the valve cap has extensions reaching down past the side of the valve stub to proximal part of the infusion cannula, wherein the extensions are provided with bearings for the spindle, and the proximal part of the infusion cannula is rotable relative to the wings around the longitudinal axis of the cannula to a position, in which the valve cap lies adjacent to one of the wings.

With the arrangement according to the invention a possibility for a single hand operation of the valve cap by injections is provided, and also the valve cap is secured against unintended opening, when the valve stub is turned down against the patient, whereby turning the valve cap is prevented by the one wing on the stationary part of the infusion cannula, as the turning movement of the cap around the axis is blocked by the wing. For opening the infusion cannula the operational staff just have to turn the infusion cannula around its longitudinal axis and swing back the valve cap distally in relation to the valve stub.

In an advangeous improvement of the infusion cannula according to the invention the skirt on its inside is provided with at least one protrusion, which, in the closed position of the cap, is positioned adjacent to the surface of the valve stub proximally to the longitudinal axis of the valve stub. In this way a dual snap-function is achieved, thereby securing that the valve cap is not freely pivotal in any of the extreme positions, as the protrusion in the other extreme position of the cap, the open one, is moved correspondingly to the other side of the surface of the valve cap distally in relation to the longitudinal axis of the valve stub.

A further advantageous improvement of the infusion cannula according to the invention is achieved by the distal part of the skirt having a length adapted to the deflection angle, whereby the skirt at deflection is positioned adjacent to the infusion cannula with a tread surface, thereby forming a stop for the turning movement. To this purpose the tread surface is formed as an oblique edge. Through this elaboration a well defined turn around the spindle is secured, what is lightening the handling of the infusion cannula.

Further advantages of the infusion cannula according to the invention is explained in the following description with reference to the drawing. In the drawing the
- fig. 1: shows a perspective view of the infusion cannula according to the invention with a closed valve cap and upright valve stub,
- fig. 2: the infusion cannula as in fig. 1, but with an opened valve cap,
- fig. 3: the infusion cannula as in fig. 1, but with a valve stub lying down,
- fig. 4: a view of the infusion cannula as seen from the proximal end with closed valve cap and upright valve stub and the lying valve stub indicated with dot-and-dash lines,
- fig. 5: a longitudinal section of the infusion cannula according to the invention, where the valve cap is shown in its two extreme positions, the open one with dot-and-dash lines, and
- fig. 6: a cross section according to the line A - A in fig. 5 through the valve stub and the valve cap on the infusion cannula according to the invention.

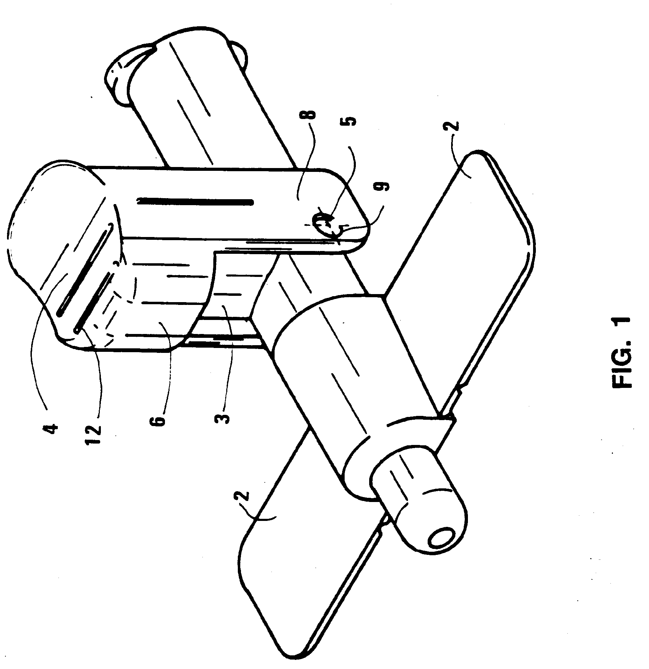

The infusion cannula 1 shown in fig. 1 is equipped with wings for the fastening onto the patient. The proximal part of the infusion cannula 1 is pivotably inerted in the distal part with the wings 2. The valve cap 4, which is shown in a closed position (pos. 1) is positioned around the spindle 5 with holes 9 serving as bearings. The valve stub 3 is just visible.

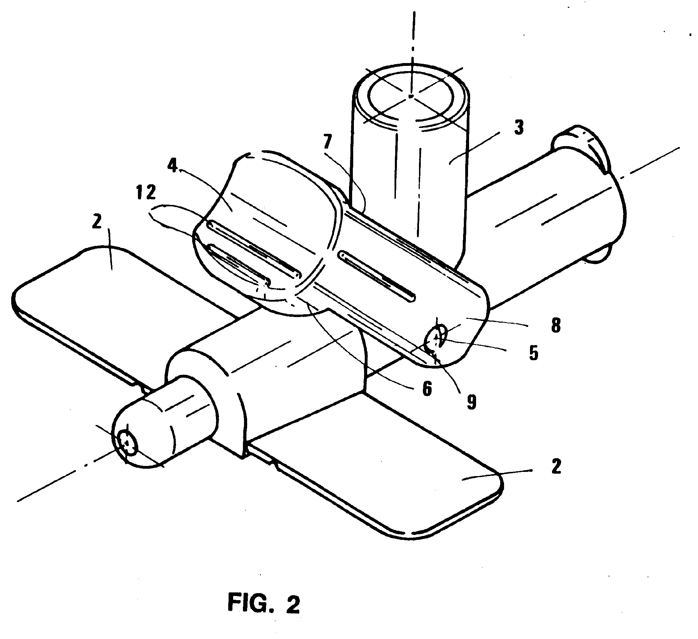

Fig. 2 shows the infusion cannula 1 with an open valve cap 4. By this there is opned for the injection syringe, which shall be introduced through the injection channel in the valve stub 3. In the open position (pos. 2) of the valve cap 4 it is treading with its tread surface 11 on the distal part of the cannula, onto which the wings 2 are fastened. The opening of the valve cap by the nursing staff can be achieved through the use of just one hand, whereby the valve cap 4 can be removed by pushing with one finger, while the rest of the hand keeps the infusion cannula itself so that no pulling or an unnecessary large pressure appears on it.

If medicin is to be administered to or examination shall be made on the patient, during which examinations contrast substances or sedating drugs or the like shall be injected, it is possible, in consideration for the patient, to avoid further inconveniences by introducing cannulas, as the nursing staff can use the valve stub 3 fastened to the infusion cannula. The injection channel and the valve mecanisms placed therein shall not be mentioned more explicitly here.

As the infusion cannula 1 generally is fastened to the backside of the patient's hand, where it is easily accessible for the nursing staff, the infusion cannula on the backside of the hand is exposed, if the patient's hand or arm is not fixed, to collisions with various hard objects in the proximity of the patient, whereby there is a risk, that the valve cap 4 is pushed off from the valve stub 3 with a contamination of the interior of the valve stub 3 as a result.

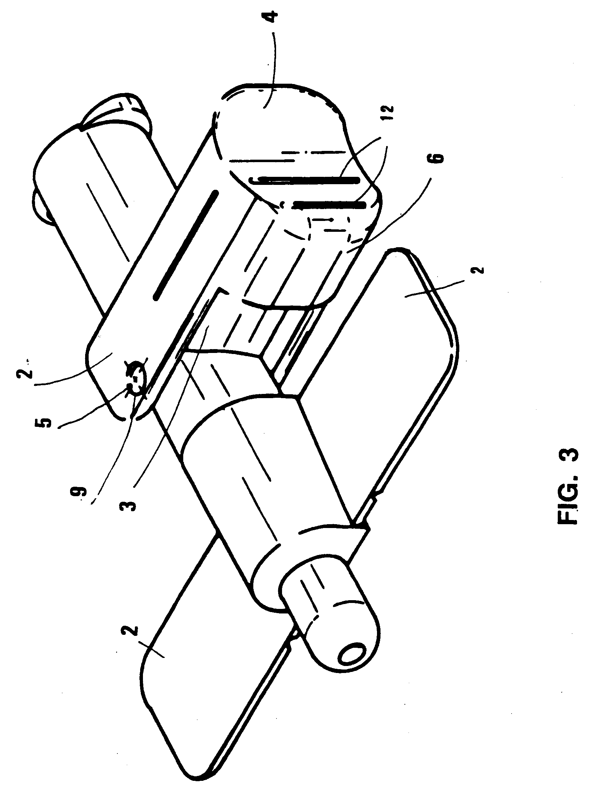

In the position of the infusion cannula shown in fig. 3 in pos. 3 the valve cap 4 is practically locked in its closed position, whereby security has been established, that the injection channel is not contaminated.

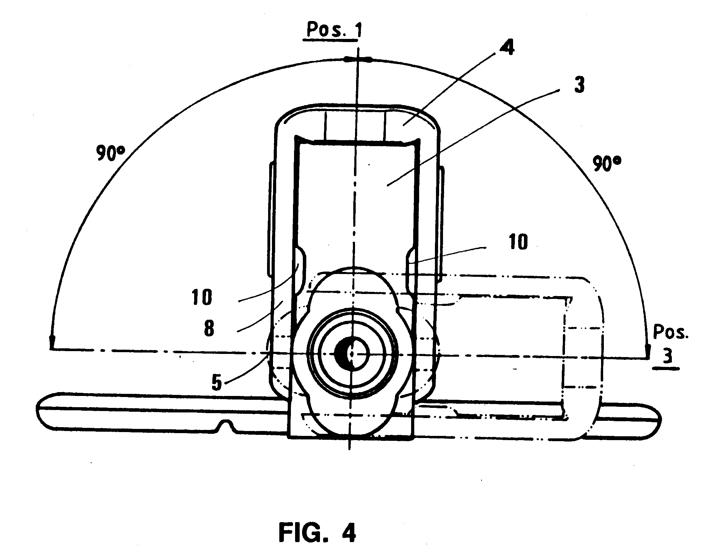

Fig. 4 shows the infusion cannula 1 as seen from the proximal end, whereby it is made clear, how the protusions 10 work together with the surface of the valve stub 3. Here it is seen, that the protrusions should not be so large, that they, at the pivotal movement of the valve cap, are lifting off the extensions 8 with the holes 9 out of intervention with the spindle 5.

Further, it is seen, how the valve stub 3 with the valve cap 4 can be laid down to pos. 3 against that part of the body, where the infusion cannula is fastened. It can thereby be seen, that the height of the infusion cannula is reduced considerably, whereby the risk of collision between the valve stub and hard objects in the proximity of the patient - and therewith the risk for an unintended removal of the valve cap 4 - is reduced drastically. It is indicated, that the valve stub 3 can be turned down to both sides, as the intended function is achieved on both sides.

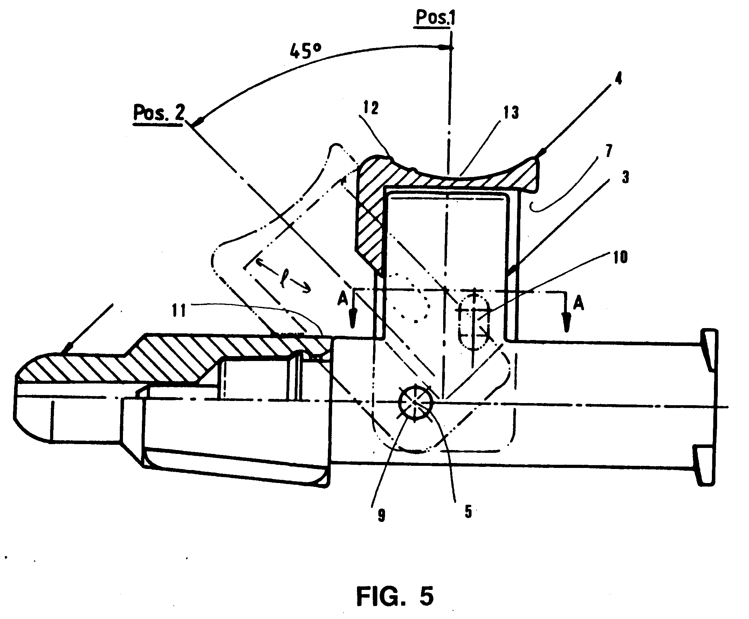

In fig. 5 a sectional view of the infusion cannula according to the invention is depicted, whereby the turning movement of the valve cap 4 is indicated. In order to ease the operation of the valve cap 4 it is, on the upper side, shaped with an in itself known depression 13, whereby a good hold for a single finger is secured.

For further securing of this hold in the depression grooves 12 are placed therein streching out parallel with the spindle 5. On the dot-and-dash shown part of the valve cap 4 it is visible, that the length λ of the skirt 6 on the distal part of the valve cap 4 is adapted to the turning angle, which here is indicated as 45°, but it could as well be any size, if just, at the turning, an admission is established to the injection channel in the valve stub 3. The under edge of this part of the skirt 6 is here oblique in order to establish a wide tread surface.

It can be seen, that the protrusions 10 in the open position of the valve cap 4 are turned to the other side of the longitudinal axis of the valve stub 3, whereby a reliable open position of the valve cap 4 is secured. Thereby further manipulation with the valve cap 4 during the injection is unnecessary, and the nursing staff can without disturbing factors finish the injection and close the valve cap 4 again and turn down the valve stub 3 along the patient's surface.

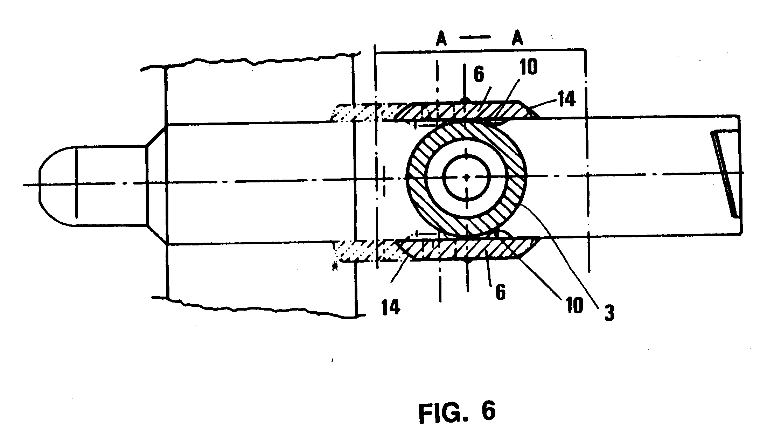

Fig. 6 indicates a sectional view through the valve stub 3 according to the line A-A in fig. 5. Here it is clearly seen, that the flanks 14 on the skirt 6 of the valve cap 4 are obliquely shaped, so that the flanks 14 can not easily catch e.g. bed linen or the like, whereby, again, a risk for contamination would be present.

## Claims

1. Infusion cannula (1) having wings (2) for fastening to a patient and at least one valve stub (3) forming an injection channel with a valve cap (4) being rotatable around two pivots extending in continuation of each other perpendicular to a plane being defined by the longitudinal axes of the cannula (1) and the valve stub (3), whereby, through the valve stub (3), injections can be made into the patient during infusion, **characterized in, that** : said pivots are provided by a spindle (5) arranged on the infusion cannula (1) on a level with the valve stub (3), and the valve cap (4) has a skirt (6) having a height approximately corresponding the height of the valve stub (3) and has an opening on the proximal side being so dimensioned, that the valve stub (3) can pass through it, when the valve cap (4) is rotated, and the skirt (6) on the sides of the valve cap (4) has extensions (8) reaching down past the side of the valve stub (3) to the proximal part of the infusion cannula (1), wherein the extensions (8) are provided with bearings (9) for the spindle (5), and the proximal part of the infusion cannula (1) is rotatable relative to the wings (2) around the longitudinal axis of the cannula (1) to a position (pos. 3) in which the valve cap (4) lies adjacent to one of the wings (2).

2. Infusion cannula according to claim 1, **characterized in, that** the skirt (6) on its inside is provided with at least one protusion (10), which, in the closed position (pos. 1) of the cap, is positioned adjacent to the surface of the valve stub (3) proximally to the longitudinal axis of the valve stub (3).

3. Infusion cannula according to claim 1 or 2, **characterized in, that** the distal part of the skirt (6) having a length (λ) adapted to the deflection angle, whereby the skirt (6) at deflection (pos. 2) is positioned adjacent to the infusion cannula (1) with a tread surface (11), thereby forming a stop for the turning movement.

4. Infusion cannula according to claim 3, **characterized in, that** the tread surface (11) is formed as an oblique edge.

5. Infusion cannula according to any of the preceding claims, **characterized in, that** the valve cap (4) on the upper side is shaped with a depression (13).

6. Infusion cannula according to claim 5, **characterized in, that** in the depression (13) grooves (12) are placed streching out parallel with the spindle (5).

7. Infusion cannula according to any of the preceding claims, **characterized in, that** the flanks (14) on the skirt (6) of the valve cap (4) are obliquely shaped.

## Patentansprüche

1. Infusionskanüle (1) mit Flügeln (2) zur Befestigung an einem Patienten und mindestens einem Ventilstutzen (3), der als ein Injektionskanal mit einer Ventilkappe (4) ausgebildet ist, die um zwei Drehzapfen drehbar ist, die sich in Verlängerung voneinander senkrecht auf eine Ebene ausdehnen, die von der Längsachse der Kanüle (1) und dem Ventilstutzen (3) begrenzt ist, wobei Injektionen während der Infusion durch den Ventilstutzen (3) in den Patienten vorgenommen werden können, **dadurch gekennzeichnet,** daß die erwähnten Drehzapfen mit einer Spindel (5) ausgerüstet sind, die auf der Infusionskanüle (1) auf der Ebene mit dem Ventilstutzen (3) angeordnet ist, und die Ventilkappe (4) eine Einfassung (6) aufweist, die eine Höhe ungefähr entsprechend der Höhe des Ventilstutzens (3) hat und auf der proximalen Seite eine Öffnung aufweist, die so dimensioniert ist, daß der Ventilstutzen (3) dadurch passieren kann, wenn die Ventilkappe (4) gedreht wird, und daß die Einfassung (6) an den Seiten der Ventilkappe (4) Verlängerungen (8) hat, die an der Seite des Ventilstutzens (3) vorbei nach unten bis zum proximalen Teil der Infusionskanüle (1) reichen, in dem die Verlängerungen (8) mit Lagern (9) für die Spindel (5) ausgerüstet sind, und der proximale Teil der Infusionskanüle (1) im Verhältnis zu den Flügeln (2) um die Längsachse der Kanüle (1) bis zu einer Stellung (Pos. 3) drehbar ist, in der die Ventilkappe (4) an einem der Flügel (2) anliegt.

2. Infusionskanüle nach Anspruch 1, **dadurch gekennzeichnet,** daß die Einfassung (6) an deren Innenseite mit mindestens einem Vorsprung (10) versehen ist, der in der geschlossenen Stellung (Pos. 1) der Kappe anliegend an der Oberfläche des Ventilstutzens (3) proximal zur Längsachse des Ventilstutzens (3) angebracht ist.

3. Infusionskanüle nach Anspruch 1 oder 2, **dadurch gekennzeichnet,** daß der distale Teil der Einfassung (6) eine an den Biegungswinkel angepaßte Länge (λ) aufweist, wobei die Einfassung (6) beim Biegen (Pos. 2) mit einer Stützfläche anliegend an der Infusionskanüle angebracht wird und dadurch eine Sperre für die Drehbewegung bildet.

4. Infusionskanüle nach Anspruch 3, **dadurch gekennzeichnet,** daß die Stützfläche (11) als eine schräge Kante ausgebildet ist.

5. Infusionskanüle nach jedem der obenerwähnten Ansprüche, **dadurch gekennzeichnet,** daß die Ventilkappe (4) auf der Oberseite mit einer Vertiefung (13) ausgebildet ist.

6. Infusionskanüle nach Anspruch 5, **dadurch gekennzeichnet,** daß parallel zur Spindel (5) verlaufende Rillen (12) in der Vertiefung (13) angeordnet sind.

7. Infusionskanüle nach jedem der obenerwähnten Ansprüche, **dadurch gekennzeichnet,** daß die Flanken (14) der Einfassung (6) der Ventilkappe (4) schräg ausgebildet sind.

## Revendications

1. Une canule de perfusion (1) possédant des ailettes (2) pour la fixation à un malade et au moins une tige de soupape (3) formant un canal d'injection, dotée d'un capuchon de soupape (4) tournant sur deux pivots placés dans le prolongement l'un de l'autre perpendiculairement à un plan défini par l'axe longitudinal de la canule (1) et de la tige de soupape (3), ce qui permet, pendant la perfusion, de donner des injections au malade à travers la tige de soupape (3), **caractérisée par le fait que** lesdits pivots sont munis d'une broche (5) montée sur la canule de perfusion (1) au niveau de la tige de soupape (3), et que le capuchon de soupape (4) comporte une jupe (6) d'une hauteur correspondant approximativement à la hauteur de la tige de soupape (3) ainsi qu'une ouverture du côté proximal dimensionnée en sorte de permettre le passage de la tige de soupape (3) lorsque le capuchon de soupape (4) est tourné, et que la jupe (6) comporte, sur les parties latérales du capuchon de soupape (4), des extensions (8) s'étendant au-delà de la partie latérale de la tige de soupape (3) jusqu'à la partie proximale de la canule de perfusion (1), ces extensions (8) étant pourvues de coussinets (9) pour la broche (5) et que la partie proximale de la canule de perfusion (1) peut effectuer un mouvement rotatif par rapport aux ailettes (2) dans l'axe longitudinal de la canule (1) jusque dans une position (pos. 3) dans laquelle le capuchon de soupape (4) est adjacent à l'une des ailettes (2).

2. Une canule de perfusion conforme à la revendication 1, **caractérisée par le fait que** la jupe (6) est pourvue sur sa face intérieure d'au moins une protubérance (10), qui, dans la position fermée (pos. 1) du capuchon, est adjacente à la surface de la tige de soupape (3) proximalement à l'axe longitudinal de la tige de soupape (3).

3. Une canule de perfusion conforme aux revendications 1 et 2, **caractérisée par le fait que** la partie distale de la jupe (6) à une longueur (λ) adaptée à l'angle de fléchissement, ce qui fait qu'au fléchissement (pos. 2), la jupe (6) est adjacente à la canule de perfusion (1), qui est pourvue d'une chape (11), ce qui arrête le mouvement rotatif.

4. Une canule de perfusion conforme à la revendication 3, **caractérisée par le fait que** la chape (11) est bisautée.

5. Une canule de perfusion conforme à chacune des revendications précédentes, **caractérisée par le fait que** le capuchon de soupape (4) comporte une concavité (13) à son sommet.

6. Une canule de perfusion conforme à la revendication 5, **caractérisée par le fait que** la concavité (13) comporte des rainures (12) s'étendant parallèlement à la broche (5).

7. Une canule de perfusion conforme à chacune des revendications précédentes **caractérisée par le fait que** les flancs (14) de la jupe (6) du capuchon de soupape (4) présentent une forme oblique.
